# EUROPÄISCHE PATENTSCHRIFT

(11) **EP 0 860 437 B1**
(45) Veröffentlichungstag und Bekanntmachung des Hinweises auf die Patenterteilung: **30.10.2002**
(21) Anmeldenummer: 98101951.6
(22) Anmeldetag: 05.02.1998
(51) Int. Cl.: C07D 251/68, D06L 3/12

(54) **Verfahren zur Herstellung von substituierten 4,4'-Diaminostilben-2,2'-disulfonsäuren**
Process of preparation of substituted 4,4'-diaministilbene-2,2'-disulfonic acid
Procédé de préparation des acides 4,4'-diaministilbènes-2,2'-disulfoniques substitués

(30) Priorität: 18.02.1997 DE 19706238
(43) Veröffentlichungstag der Anmeldung: 26.08.1998
(73) Patentinhaber: BAYER AG, 51368 Leverkusen (DE)
(72) Erfinder: Feldhues, Ulrich, Dr., 29412 Charleston S.C. (US); Brockmann, Rolf, Dr., 51469 Bergisch Gladbach (DE); Eckstein, Udo, Dr., 51061 Köln (DE); Szeymies, Detlef, Dr., 51515 Kürten (DE)

(56) Entgegenhaltungen:
- EP-A- 0 626 374
- WO-A-96/00220
- DE-C- 19 500 791
- FR-A- 2 611 365

## Beschreibung

Die Erfindung betrifft ein verbessertes Verfahren zur Herstellung von substituierten 4,4'-Diaminostilben-2,2'-disulfonsäuren sowie die Verwendung so hergestellter Produkte als optische Aufheller.

Es ist bereits aus DE-A 19 500 791 bekannt, speziell substituierte 4,4'-Diaminostilben-2,2'-disulfonsäuren durch Umsetzung von Verbindungen der Formel 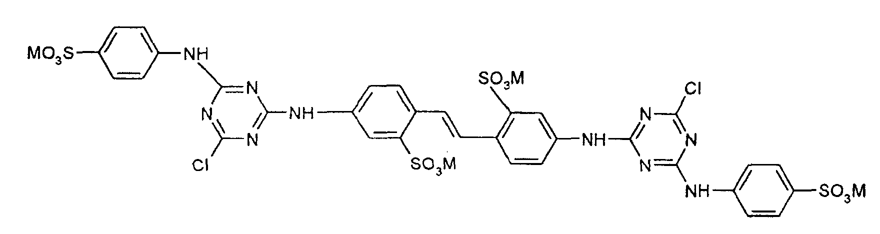 worin
- M: für ein Alkalimetallion oder ein gegebenenfalls substituiertes Ammoniumion steht,
mit 2 Äquivalenten eines speziellen Amins in Gegenwart eines Säurefängers herzustellen.

Nachteilig bei der dort beschriebenen Verfahrensvariante ist jedoch, daß noch bevor die gewünschte Reaktionstemperatur erreicht wird, es häufig zu einer Verdickung der Reaktionsmischung kommt, wodurch die Rührbarkeit beeinträchtigt wird. Dabei werden verwendete Rührer und deren Antriebe besonders stark belastet.

Aufgabe der vorliegenden Erfindung war es daher, ein Verfahren bereitzustellen, das die genannten Nachteile nicht mehr besitzt.

Es wurde nun ein Verfahren zur Herstellung von Verbindungen der Formel (I) gefunden, 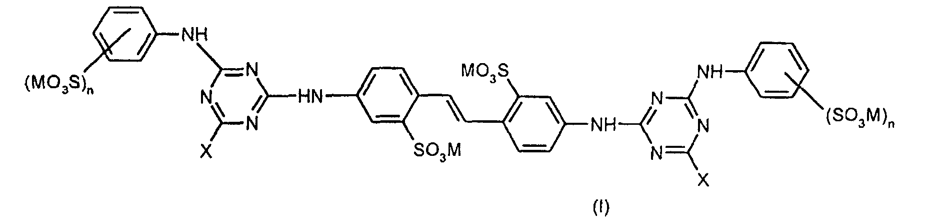 worin
- n: für 0, 1 oder 2 steht,
- M: für Wasserstoff-, ein Alkalimetallion oder ein gegebenenfalls substituiertes Ammoniumion und
- X: für Anilino, N-Alkylamino oder N,N-Dialkylamino steht, wobei die gegebenenfalls substituierten Alkylreste mit N-Alkylamino und N,N-Dialkylamino gegebenfalls durch ein Heteroatom aus der Reihe O, N oder S unterbrochen sind und im Fall von N,N-Dialkyl die beiden Alkylreste gegebenenfalls gemeinsam mit dem N-Atom, an das sie gebunden sind, einen gesättigten 5- oder 6-gliedrigen Heterocyclus bilden,
durch Umsetzung einer Verbindung der Formel (IV) 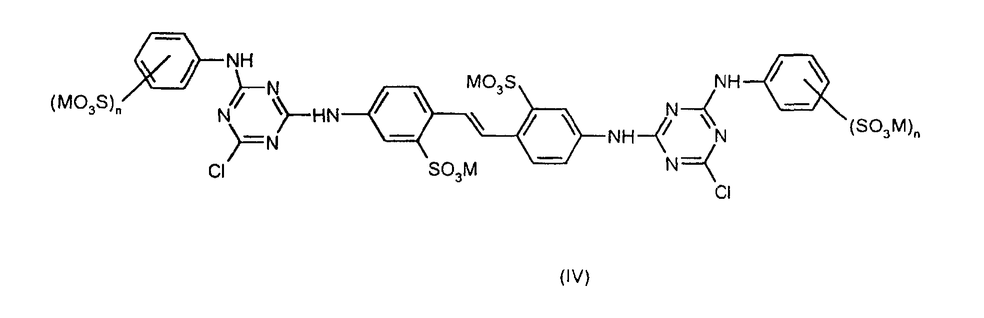 worin M und n die obengenannte Bedeutung haben,
mit einem Amin der Formel (V)

X-H (V)

worin X die obengenannte Bedeutung besitzt,
bei einem pH-Wert von 5 - 10, gegebenenfalls in Gegenwart eines von V verschiedenen Säurefängers, dadurch gekennzeichnet, daß man einem wäßrigen Reaktionsmedium mit einer Temperatur von wenigstens 40°C die Verbindung der Formel (IV) zugibt und daß man das Amin der Formel (V) und gegebenenfalls den Säurefänger unabhängig voneinander vor und/oder während der Zugabe von IV dem wäßrigen Reaktionsmedium zugibt.

In einer bevorzugten Ausführungsform des erfindungsgemäßen Verfahrens steht
- M: für Na-, oder K-Ionen, NH₄⁺ oder Triethanolammonium,
- n: steht insbesondere für Null.

Die Alkylreste im N-Alkylamino und N,N-Dialkylamino sind bevorzugt C₁-C₄-Alkylreste, die gegebenenfalls durch ein O-Atom unterbrochen sind. Als mögliche Substituenten der Alkylreste sind beispielsweise die in der Chemie der Weißtöner üblichen Substituenten wie Hydroxy, Cyano, Carbamoyl oder Sulfo zu nennen.

Beispiele für gesättigte 5- und 6-gliedrige Heteroyclen, die aus den beiden Alkylresten der N,N-Dialkylaminogruppe und dem Stickstoffatom, an das sie gebunden sind, gebildet werden können, sind beispielsweise Pyrrolidin, Piperidin, N-Methylpiperazin, N-(2-Hydroxyethyl)piperazin und insbesondere Morpholin.

Das wäßrige Reaktionsmedium besitzt vorzugsweise eine Temperatur von wenigstens 60°C, insbesondere von 60 bis 140°C, besonders bevorzugt 80 bis 100°C. Während der Zugabe von IV zum Reaktionsmedium wird die Temperatur des Reaktionsmediums vorzugsweise konstant gehalten. Für Temperaturen, die oberhalb des Siedepunktes der Reaktionsmischung liegen, erfolgt die Umsetzung vorzugsweise unter dem Eigendruck des wäßrigen Reaktionsmedium bis 6 bar.

Die Temperatur des Reaktionsmediums liegt vorzugsweise um 20°C, insbesondere um 40°C höher als die der zuzugebenden Verbindung IV.

Die Umsetzung von IV mit V erfolgt vorzugsweise bei einem pH-Wert von 6 bis 9, insbesondere bei 7 bis 8.

Die Menge des Amins V beträgt vorzugsweise 2 oder mehr Mol-Äquivalente, bezogen auf die Verbindung der Formel (IV). Erfolgt das erfindungsgemäße Verfahren ohne einen von V verschiedenen Säurefänger, so beträgt sie vorzugsweise 4 bis 5 Mol Äquivalente.

Besonders bevorzugt wird das Amin V in einer Menge von 2 Mol-Äquivalenten zuzüglich eines 10 - 70 %igen molaren Überschuß zusammen mit einem von V verschiedenen Säurefänger eingesetzt.

Bevorzugte Amine der allgemeinen Formel V sind Morpholin, Methylamin, Ethylamin, Anilin, 2-Hydroxyethylamin, 2-Hydroxypropylamin und insbesondere Di-(2-hydroxyethyl)amin und Di-(2-hydroxypropyl)amin.

Geeignete Säurefänger sind wasserlösliche, säurebindende Mittel wie beispielsweise Alkalihydroxide wie NaOH oder KOH, Alkalicarbonate, wie Na₂CO₃ oder K₂CO₃, Alkalihydrogencarbonate wie NaHCO₃ oder KHCO₃ oder tert.-Amine wie Triethanolamin. Diese können in fester Form, beispielsweise mittels einer Förderschnecke oder bevorzugt in Form ihrer wäßrigen Lösungen eingesetzt werden.

Bevorzugte Säurefänger sind Na₂CO₃, K₂CO₃, NaOH oder KOH, insbesondere in Form ihrer wäßrigen Lösungen.

Die Zugabe des Säurefängers erfolgt bevorzugt separat. Der Säurefänger kann bereits ganz oder anteilig in Lösung oder Suspension von IV enthalten sein oder ganz oder anteilig im wäßrigen Reaktionsmedium vorgelegt sein.

Bevorzugt erfolgt die Zugabe des Säurefängers pH-gesteuert. Insbesondere wird der Säurefänger in Abhängigkeit vom pH-Wert automatisch zudosiert.

Bei der pH-gesteuerten Zugabe des Säurefangers dient der aus dem pH-Bereich von 5 bis 10 ausgewählte pH-Wert als Steuergröße. Wird dieser Sollwert unterschritten, fließt solange Säurefänger in die vorzugsweise gerührte Reaktionsmischung ein, bis der ursprüngliche Wert wieder erreicht ist. Das kann manuell oder vorzugsweise automatisch mit Hilfe eines Titrators erfolgen. Als Titrator eignet sich beispielsweise ein DULCOMETER® vom Typ PR F2K2 der Firma Pro Minent®.

Die Abweichung vom pH-Sollwert beträgt bei dieser Verfahrensvariante in der Regel nicht mehr als -0,5 oder +0,3 pH-Einheiten. Wenn ( IV) verbraucht ist und somit unter diesen Bedingungen keine weitere Salzsäure mehr entsteht, stellt sich der als Sollwert gewählte pH-Wert wieder ein, ohne noch einmal wieder abzusinken, und der Zufluß stoppt.
Praktisch ist der Endpunkt für die Zugabe des Säurefängers bereits dann erreicht, wenn innerhalb von 10 Minuten weniger als 0,5 % der berechneten Menge an IV verbraucht werden.

Das wäßrige Reaktionsmedium ist vorzugsweise Wasser, das gegebenenfalls schon vor der Zugabe der Verbindung IV das Amin V anteilig oder vollständig enthält.

Die Verbindung der Formel (IV) kann dem Reaktionsmedium in fester Form oder bevorzugt als wäßrige Lösung oder Suspension zugegeben werden.

Wird die Verbindung der Formel (IV) als wäßrige Lösung oder Suspension verwendet, so können auch Tenside wie beispielsweise alkoxylierte Fettalkohole zugegen sein.

In einer bevorzugten Ausführungsform des erfindungsgemäßen Verfahrens enthält die wäßrige Lösung oder Suspension von IV bereits das Amin V ganz oder anteilig, und besitzt vorzugsweise einen pH-Wert, bei dem das Amin V überwiegend in Form seiner protonierten Form der Formel VI vorliegt

H-X-H^{⊕} (VI),

worin
X die obengenannte Bedeutung besitzt. Dieser pH-Wert ist vom pK-Wert des jeweiligen Amins abhängig und beträgt vorzugsweise 4 bis 10, insbesondere 4-8.

Besonders bevorzugt wird die Verbindung der Formel (IV) in Form einer wäßrigen Lösung oder Suspension eingesetzt, die 1.8 bis 2.7-Mol-Äquivalente Amin V in überwiegend protonierter Form VI enthält, eingesetzt. Der pH-Wert, bei dem mehr als 50 % des Amins in protonierter Form vorliegen, ist selbstverständlich vom pK-Wert des Amins abhängig; vorzugsweise liegt der pH-Wert bei 4 bis 10, insbesondere 4 bis 8. Dem wäßrigen Reaktionsmedium werden für diesen Fall gegebenenfalls bis 0,7 Mol-Äquivalente des Amin V, bezogen auf IV, vor, während oder nach der Zugabe der wäßrigen Lösung oder Suspension von IV zugegeben.

Bevorzugt wird eine Verbindung der Formel IV in Form ihrer Syntheselösung oder -suspension verwendet.

Die Herstellung von IV erfolgt dabei vorzugsweise entweder durch Umsetzung von einer 4,4'-Diaminostilben-2,2'-disulfonsäure der Formel (II) 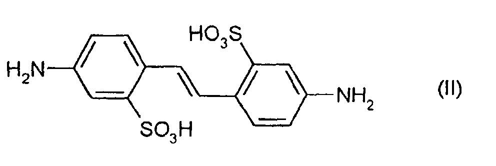 mit 2 Mol-Äquivalenten Cyanurchlorid, bezogen auf II und anschließender Umsetzung mit 2 Mol-Äquivalenten eines Anilins der Formel (III) 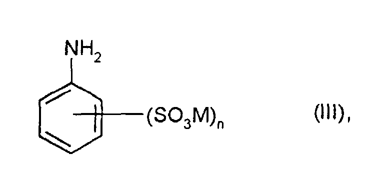 worin
n und M die oben angegebene Bedeutung haben oder durch Umsetzung von Cyanurchlorid mit 1 Mol-Äquivalent, bezogen auf Cyanurchlorid, eines Anilins der Formel (III) und anschließender Umsetzung mit 1/2 Mol-Äquivalent einer Verbindung der Formel (II).

Die Angabe der Mol-Äquivalente der zur Herstellung der Verbindung IV eingesetzten Reaktanden dient lediglich zur Angabe der Stöchiometrie und schließt nicht größere oder kleinere Mengen aus, die gegebenenfalls technisch sinnvoller sind.

Bei der Herstellung der Verbindung IV wird beispielsweise in einer ersten Stufe ein Dialkalisalz der 4,4'-Diaminostilben-2,2'-disulfonsäure der Formel (II) in Form einer wäßrigen Lösung einer wäßrigen Cyanurchlorid-Suspension bei einer Temperatur von 0 bis 25 °C und einem pH-Wert von 3,5 bis 5,5 in Anwesenheit oder unter gleichzeitiger Zugabe eines säurebindenden Mittels, vorzugsweise eines Alkalicarbonats wie Na₂CO₃ oder K₂CO₃ oder eines Alkalihydrogencarbonats wie NaHCO₃ oder KHCO₃, zur Neutralisation der freigesetzten Salzsäure zugegeben.

Die wäßrige Cyanurchlorid-Suspension enthält üblicherweise 0,05 - 2 Gew.-% (bezogen auf Cyanurchlorid) eines benetzenden Tensids, vorzugsweise aus der Reihe alkoxylierter Fettalkohole, insbesondere einen oder mehrere C₈-C₁₄-Fettalkohole mit 3 bis 10 Mol Ethylenoxid und 0 bis 25 Mol Propylenoxid, z.B. den Polyether aus Laurylalkohol und 5 Mol Ethylenoxid und/oder den Polyether aus einem C₁₀-Alkohol und 6 Mol Ethylenoxid und 8 Mol Propylenoxid.

Weiterhin kann die Cyanurchlorid-Suspension auch 0,05 bis 2,0 Gew.% (bezogen auf Cyanurchlorid) eines Entschäumers enthalten, der z.B. etwa zur Hälfte aus einer Mischung von C₁₅-Alkansulfamid und Ammoniumsalz der C₁₅-Alkansulfonsäure und zur anderen Hälfte aus dem entsprechenden C₁₅-Alkan besteht.

Eine bevorzugte Ausführungsform des erfindungsgemäßen Verfahrens besteht darin, die Zugabe der Lösung von (II), die zusätzlich eine zur Neutralisation der bei der Reaktion entstehenden Salzsäure gerade ausreichende Menge eines wasserlöslichen säurebindenden Mittels enthält, zur wäßrigen Cyanurchlorid-Suspension pH-gesteuert bei einem pH-Sollwert aus dem Bereich 3,5 - 5,5, vorzugsweise 4,0 - 5,0, bei einer Temperatur von 5 bis 20 °C durchzuführen, wobei auch hier die Abweichung des Sollwertes in der Regel nicht mehr als -0,5 oder + 0,3 pH-Einheiten beträgt. Praktisch ist der Endpunkt für die Zugabe der Lösung von (II) auch hier bereits dann erreicht, wenn in 10 Minuten weniger als 0,5 % Lösung von (II) verbraucht werden.

In einem zweiten Schritt läßt man bei dieser Verfahrensvariante die Verbindung (III) zulaufen und hält die Temperatur vorzugsweise auf 10 - 60°C, insbesondere auf 20-40°C und den pH-Wert vorzugsweise bei 3,5 bis 7,5 durch Titration mit einem Säurefänger.

In einer bevorzugten Ausführungsform zur Herstellung der Verbindung IV wird in diesem zweiten Schritt als Säurefänger das Amin XH (V) verwendet.

Eine weitere bevorzugte Ausführungsform zur Herstellung der Verbindung IV besteht darin, in einem ersten Schritt Verbindungen der allgemeinen Formel (III) zur wäßrigen Cyanurchlorid-Suspension einzudosieren und dabei den pH-Wert durch Zugabe von Säurefänger konstant (vorzugsweise auf 1.5 bis 6.0) zu halten. Besonders vorteilhaft ist es, wenn eine Lösung oder Suspension von (III) zusätzlich eine zur Neutralisation der bei der Reaktion entstehenden Salzsäure gerade ausreichende Menge eines wasserlöslichen säurebindenden Mittels enthält, und man dann diese Lösung oder Suspension von (III) gegebenenfalls pH-gesteuert zur wäßrigen Cyanurchlorid-Suspension eindosiert.

In einem zweiten Reaktionsschritt ist es besonders vorteilhaft, die Zugabe einer Lösung von (II), die zusätzlich eine zur Neutralisation der bei der Reaktion entstehenden Salzsäure gerade ausreichende Menge eines wasserlöslichen säurebindenden Mittels enthält, zur wäßrigen Reaktions-Lösung oder -Suspension des ersten Schrittes pH-gesteuert bei einem pH-Sollwert von 3,5 - 7,0, vorzugsweise von 4,5 - 6,0, bei einer Temperatur von 25 bis 90 °C durchzuführen, wobei auch hier die Abweichung des Sollwertes in der Regel nicht mehr als -0,5 oder + 0,3 pH-Einheiten beträgt. Praktisch ist der Endpunkt für die Zugabe der Lösung von (II) auch hier bereits dann erreicht, wenn in 10 Minuten weniger als 0,5 % Lösung von (II) verbraucht werden.

Das erfindungsgemäße Verfahren ist besonders bevorzugt zur Herstellung der Verbindungen der Formel (I) in technischem Maßstab. Insbesondere für Reaktionsvolumina von 10 bis 100 m³.

Die Aufarbeitung der nach dem erfindungsgemäßen Verfahren hergestellten Verbindungen der allgemeinen Formel (I) kann in bekannter Weise z.B. durch Aussalzen und Abfiltrieren erfolgen, oder man fällt (I) in bekannter Weise als in Wasser schwerlösliche freie Säure (M =H) aus der warmen Lösung aus und filtriert sie ab.

Der feuchte Kristallkuchen kann dann in bekannter Weise zur Herstellung einer lagerstabilen flüssigen Einstellung gegebenenfalls unter Zusatz eines oder mehrerer Formulierhilfsmittel, wie nichtionische oder anionische Tenside und/oder polare organische Lösungsvermittler wie Polyglykole oder Harnstoff, in Wasser gelöst werden, wobei man im Falle der freien Säure soviel Base zusetzt, daß diese wieder in ein gut wasserlösliches Salz zurückverwandelt wird. Eine ebenfalls bekannte, einfache, kostengünstige Aufarbeitung besteht darin, die Rohlösung von (I) durch Membrantrennverfahren unter Druck (Druckpermeation) weitgehend zu entsalzen, aufzukonzentrieren und direkt in die handelsübliche stabile Flüssigeinstellung zu überführen, z.B. nach DE-C 32 34 784 oder DE-A 22 04 725 oder ggf zu trocknen, insbesondere zu sprühtrocknen.

Diese rationelle Aufarbeitungsmethode läßt sich um so erfolgreicher anwenden, je reiner die Aufheller-Rohlösungen sind, die das Verfahren liefert, da die Nebenprodukte und Verunreinigungen bei der Druckpermeation mit Ausnahme der niedermolekularen Anteile nicht mit dem Permeat ausgeschleust werden, somit letztlich im Aufhellerpräparat verbleiben und dessen Effizienz beeinträchtigen können.

Hier bietet das erfindungsgemäß verbesserte Verfahren gegenüber den bisher bekannten Verfahren erhebliche Vorteile; denn es liefert die Aufheller-Rohlösungen ohne zusätzliche Reinigungsschritte in einer bisher nicht erreichten Reinheit und mit einer erheblich verringerten Abwasserlast für den Anwender. Bei der herkömmlichen Aufarbeitung durch Aussalzen oder Ausfällen in der sauren Form entsteht auch für den Hersteller eine viel geringere Abwasserlast als durch die bisherigen Verfahren.

Die nach dem erfindungsgemäßen Verfahren hergestellten Verbindungen der Formel (I) sind wichtige optische Aufheller u.a. für Polyamid, Cellulose, Papier und Waschmittel.

### Beispiele

### Beispiel 1

In einen Reaktor A wurden 700 ml Wasser und 10 g Kochsalz eingetragen und 10 min verrührt. Anschließend setzte man unter Rühren 1,0 g eines Polyethers aus Isodecylalkohol, 6 mol Ethylenoxyd und 8 mol Propylenoxyd zu und kühlte auf ca. 10°C ab.

Man trug unter Rühren 100 g Cyanurchlorid (0,542 mol) ein, spülte mit 100 ml Wasser nach und verrührte die Suspension, bis der pH auf 4,5 gefallen war.

Dazu titrierte man eine wäßrige auf 10°C-gekühlte Lösung, die in 1200 ml 0,3 mol 4,4'-Diaminostilben-2,2'-disulfonsäure-dinatriumsalz und 0,3 mol Natriumcarbonat enthielt, wobei man die Temperatur der Reaktionsmischung auf 18°C ansteigen ließ. Für die Zugabe verwendete man einen automatischen Titrator, den man auf den oberen Grenzwert von pH 4,5 einstellte.

Theoretisch konnten 1084 ml verbraucht werden. Der Endpunkt der Reaktion war erreicht, als innerhalb von 10 Minuten weniger als 5 ml verbraucht wurden. Das war nach 2 bis 2,5 Stunden bei einem Verbrauch von 99 % der Theorie der Fall. Es entstand eine gutrührbare hellgelbe Suspension.

Man wechselte die Titratorvorlage. Die Titratorvorlage enthielt 87,2 g 80 %ige wäßrige Diethanolaminlösung (0,664 mol). Man rührte den Inhalt des Reaktor A weitere 30 min bei pH 4,5 nach. In dieser Zeit wurde ca. 1 g 80 %iger wäßriger Diethanolaminlösung aufgenommen.

Anschließend ließ man 500 ml Natriumsulfanilatlösung, die in diesen 500 ml 0,528 mol Natriumsulfanilat enthielt, innerhalb von 30 Minuten in den Reaktor A einlaufen, wobei man die Temperatur im Reaktor A auf 35°C erhöhte. Man rührte 2 Stunden bei 35°C nach. Bis zu diesem Zeitpunkt wurden über den Titrator ca. 74 g 80 %ige wäßrige Diethanolaminlösung (0,56 mol) eindosiert. Die Aufnahme betrug am Ende der Nachrührzeit weniger als 2 ml in 10 Minuten.

Man ließ den Rest der wäßrigen Diethanolaminlösung zulaufen.

In einem zweiten Reaktor B legte man 300 ml Wasser vor und erwärmte auf 95°C.

Innerhalb von 1 Stunde wurde der Inhalt aus dem Reaktor A in den Reaktor B überführt. Dabei wurde die Temperatur im Reaktor B auf 95°C und der pH auf 7,5 durch Titration mit einer 15%igen Sodalösung konstant gehalten. Anschließend wurde unter fortgesetzter Titration 1 Stunde auf 100°C erhitzt.

Man erhielt 3,4 kg einer wäßrigen Rohlösung mit einer spezifischen Extinktion von 49 bei 350 nm. Die Rohlösung enthielt 3,2 % (0,11 kg) Kochsalz und 9 % (0,31 kg) Aktivsubstanz, die der Verbindung der Formel 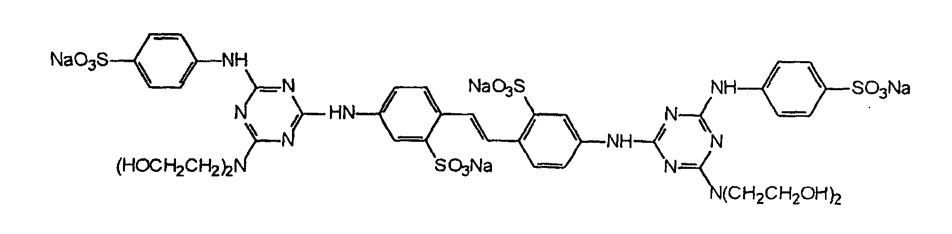 entspricht.

Die Rohlösung wurde in der in DE-C 32 34 784, Beispiel 2 angegebenen Weise durch Druckpermeation bis auf einen Restgehalt von 0,5 % Kochsalz entsalzen und dann auf ein Gewicht von 1,34 kg aufkonzentriert. Man erhielt eine Flüssigeinstellung mit einer spezifischen Extinktion von 125 bei 350 nm, 23 % Aktivsubstanz und einem Elektrolytgehalt von <0,5 %.

### Beispiel 2

Man wiederholte Beispiel 1 mit dem Unterschied, daß man als Säurefänger im Reaktor B 10%ige Natronlauge einsetzte und den Rest der wäßrigen Diethanolaminlösung in den Reaktor B eintrug. Die Aufarbeitung erfolgte analog zu Beispiel 1. Man erhielt 1,34 kg einer Flüssigeinstellung mit einer spezifischen Extinktion von 125 bei 350 nm.

### Beispiel 3

In einem Reaktor A wurden 700 ml Wasser und 10 g Kochsalz eingetragen und 10 min verrührt. Anschließend setzte man unter Rühren 1,0g eines Polyethers aus Isodecylalkohol, 6 mol Ethylenoxyd und 8 mol Propylenoxyd zu und kühlte auf ca. 10°C ab.

Man trug unter Rühren 100 g Cyanurchlorid (0,542 mol) ein, spülte mit 100 ml Wasser nach und verrührte die Suspension, bis der pH auf 4,5 gefallen war.

Dazu titrierte man eine wäßrige auf 10°C-gekühlte Lösung, die in 1200 ml 0,3 mol 4,4'-Diaminostilben-2,2'-disulfonsäure-dinatriumsalz und 0,3 mol Natriumcarbonat enthielt, wobei man die Temperatur der Reaktionsmischung auf 18 °C ansteigen ließ. Für die Zugabe verwendete man einen automatischen Titrator, den man auf den oberen Grenzwert von pH 4,5 einstellte.

Theoretisch konnten 1084 ml verbraucht werden. Der Endpunkt der Reaktion war erreicht, als innerhalb von 10 Minuten weniger als 5 ml verbraucht werden. Das war nach 2 bis 2,5 Stunden bei einem Verbrauch von 99 % der Theorie der Fall. Es entstand eine gutrührbare hellgelbe Suspension.

Man setzte 25 g einer 20 %igen Kochsalzlösung zu.

Man wechselte die Titratorvorlage. Die Titratorvorlage enthielt 87,2 g einer 80 %igen, wäßrigen Diethanolaminlösung (0,664 mol). Man rührte den Inhalt des Reaktor A weitere 30 min bei pH 5,5 nach.

In dieser Zeit wurde ca. 1 g 80 %iger wäßriger Diethanolaminlösung aufgenommen.

Anschließend ließ man 50,2 g Anilin (0,54 mol) innerhalb von 30 Minuten einlaufen, wobei man die Temperatur im Reaktor auf 25°C ansteigen ließ. Man rührte 1,5 Stunden bei 25°C nach. Bis zu diesem Zeitpunkt wurden über den Titrator ca. 71 g einer 80 %igen wäßrigen Diethanolaminlösung (0,54 mol) eindosiert. Die Aufnahme betrug am Ende der Nachrührzeit weniger als 2 ml in 10 Minuten.

In einem zweiten Reaktor B legte man 300 ml Wasser vor, gab den Rest der Diethanolaminlösung (16,2 g) zu und erwärmte auf 95°C.

Innerhalb von 1,5 Stunden wurde der Inhalt aus dem Reaktor A in den Reaktor B überführt. Dabei wurde die Temperatur im Reaktor B auf 95°C und der pH auf 7,5 durch Titration mit 10%iger Natronlauge konstant gehalten. Anschließend wurde unter fortgesetzter Titration 2,5 Stunden auf 98 - 100°C erhitzt.

Man ließ auf 85°C abkühlen und stellte bei dieser Temperatur durch Zugabe von Salzsäure einen pH von 4,2 ein. Man rührte 30 min nach und ließ dabei auf 50 -55°C abkühlen.

Das Produkt wurde abfiltriert und sorgfältig mit Wasser gewaschen. Nach dem Trocknen bei 50°C im Vakuum erhielt man 245g Produkt.

Das Produkt entspricht der Verbindung der Formel

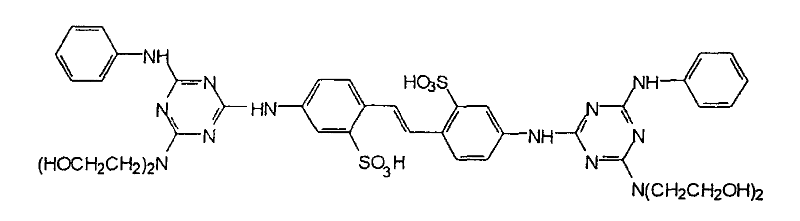

### Beispiel 4

In einem Reaktor A wurden 700 ml Wasser und 10 g Kochsalz eingetragen und 10 min verrührt. Anschließend setzte man unter Rühren 1,0 g eines Polyethers aus Isodecylalkohol, 6 mol Ethylenoxyd und 8 mol Propylenoxyd zu und kühlte den Ansatz auf ca. 10 °C ab.

Man trug unter Rühren 100 g Cyanurchlorid (0,542 mol) ein, spülte mit 100 ml Wasser nach und verrührte die Suspension, bis der pH auf 4,5 gefallen war.

Dazu titrierte man eine wäßrige auf 10°C-gekühlte Lösung, die in 1200 ml 0,3 mol 4,4'-Diaminostilben-2,2'-disulfonsäure-dinatriumsalz und 0,3 mol Natriumcarbonat enthielt, wobei man die Temperatur der Reaktionsmischung auf 18°C ansteigen ließ. Für die Zugabe verwendete man einen automatischen Titrator, stellte diesen auf den oberen Grenzwert von pH 4,5 ein.

Theoretisch konnten 1084 ml verbraucht werden. Der Endpunkt der Reaktion war erreicht, als innerhalb von 10 Minuten weniger als 5 ml verbraucht wurden. Das war nach 2 bis 2,5 Stunden bei einem Verbrauch von 99 % der Theorie der Fall. Es entstand eine gutrührbare hellgelbe Suspension.

Man setzte 100 g einer 20 %igen Kochsalzlösung zu.

Man wechselte die Titratorvorlage. Die Titratorvorlage enthielt 10 %ige wäßrige Natronlaugelösung. Man rührte den Inhalt des Reaktor A weitere 30 min bei pH 6,5 nach.

Anschließend ließ man 50,2 g Anilin (0,54 mol) innerhalb 30 Minuten einlaufen, wobei man die Temperatur im Reaktor auf 30°C ansteigen ließ. Man rührte 1 Stunde bei 30°C nach. Dafür wurden über den Titrator ca. 216 g 10%ige wäßrige Natronlaugelösung (0,54 mol) eindosiert. Die Aufnahme betrug am Ende der Nachrührzeit weniger als 2 ml in 10 Minuten.

In einem zweiten Reaktor B legte man 300 ml Wasser vor, gab 56,6 g Morpholin (0,65 mol) zu und erwärmte auf 95°C.

Innerhalb von 1 Stunde wurde der Inhalt aus dem Reaktor A in den Reaktor B überführt. Dabei wurde die Temperatur im Reaktor B auf 95°C und der pH auf 7,5 durch Titration mit 10%iger Natronlauge konstant gehalten. Anschließend wurde unter fortgesetzter Titration 1 Stunde bei pH 8,5 auf 95 - 100°C erhitzt. Danach wurde der Ansatz für 1h auf 135°C erhitzt.

Man ließ auf 90°C abkühlen und setzte 100 g Natronlauge 50%ig zu. Der Ansatz wurde über eine Nutsche abgesaugt und sorgfältig mit heißer verdünnter Kochsalzlösung gewaschen.

Nach dem Trocknen bei 100°C im Vakuum erhielt man 260 g Produkt.

Das Produkt entspricht der Verbindung der Formel

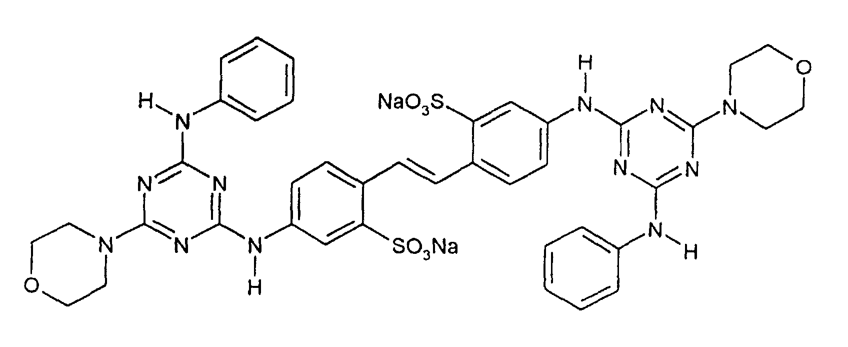

### Beispiel 5

In einem Reaktor A wurden 700 ml Wasser und 10 g Kochsalz eingetragen und 10 min verrührt. Anschließend setzte man unter Rühren 1,0 g eines Polyethers aus Isodecylalkohol, 6 mol Ethylenoxyd und 8 mol Propylenoxyd zu und kühlte den Ansatz auf ca. 10°C ab.

Man trug unter Rühren 100 g Cyanurchlorid (0,542 mol) ein und spülte mit 100 ml Wasser nach und verrührte die Suspension, bis der pH auf 4,5 gefallen war.

Dazu titrierte man eine wäßrige auf 10°C-gekühlte Lösung, die in 1200 ml 0,3 mol 4,4'-Diaminostilben-2,2'-disulfonsäure-dinatriumsalz und 0,3 mol Natriumcarbonat enthielt, wobei man die Temperatur der Reaktionsmischung auf 18°C ansteigen ließ. Für die Zugabe verwendete man einen automatischen Titrator und stellte diesen auf den oberen Grenzwert von pH 4,5 ein.

Theoretisch konnten 1084 ml verbraucht werden. Der Endpunkt der Reaktion war erreicht, als innerhalb von 10 Minuten weniger als 5 ml verbraucht wurden. Das war nach 2 bis 2,5 Stunden bei einem Verbrauch von 99 % der Theorie der Fall. Es entstand eine gutrührbare hellgelbe Suspension.

Man setzte 100 g einer 20 %igen Kochsalzlösung zu.

Man wechselte die Titratorvorlage. Die Titratorvorlage enthielt 10 %ige Natronlauge Man rührte den Inhalt des Reaktor A weitere 30 min bei pH 6,5 nach.

Anschließend ließ man 50,2 g Anilin (0,54 mol) innerhalb von 30 Minuten einlaufen, wobei man die Temperatur im Reaktor auf 30°C ansteigen ließ. Man rührte 1 Stunde bei 30°C nach. Dafür wurden über den Titrator ca. 216 g 10%iger Natronlauge (0,54 mol) eindosiert. Die Aufnahme betrug am Ende der Nachrührzeit weniger als 2 ml in 10 Minuten.

Man setzte 67,2 g Methylaminlösung 30%ig (0,65 mol) zu.

In einem zweiten Reaktor B legte man 300 ml Wasser vor, und erwärmte auf 95°C.

Innerhalb von 1 Stunde wurde der Inhalt aus dem Reaktor A in den Reaktor B überführt. Dabei wurde die Temperatur im Reaktor B auf 95°C und der pH auf 7,5 durch Titration mit einer 15%igen Sodalösung konstant gehalten. Anschließend wurde unter fortgesetzter Titration 1 Stunde bei pH 8,5 auf 95-100°C erhitzt. Danach wurden 100 g Natronlauge 50%ig zugesetzt. Unter Einleiten von Stickstoff ließ man das überschüssige Methylamin entweichen.

Nach der Sprühtrocknung erhielt man 215 g Produkt.

Das Produkt entspricht der Verbindung der Formel

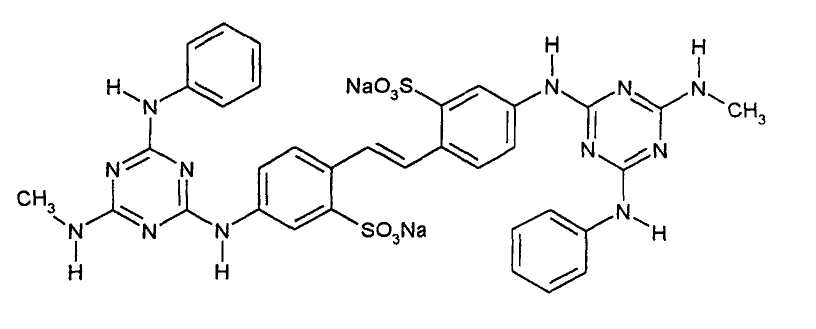

### Beispiel 6

In einem Reaktor A befand sich eine Lösung aus 167 g 4,4'-Bis[(4-(2,5disulfo)anilino-6-chlor-1,3,5-triazin-2-yl)amino]-stilben-2,2'-disulfonsäurehexanatriumsalz in 1275 ml Wasser.

In einem zweiten Reaktor B legte man 200 ml Wasser und 44 (0.335 mol) Diethanolaminlösung (80 %ig) vor, und erwärmte auf 95°C.

Innerhalb von 1 Stunde wurde der Inhalt aus dem Reaktor A in den Reaktor B überführt. Dabei wurde die Temperatur im Reaktor B auf 95°C gehalten und der pH auf 7,5 durch Titration mit einer 15%igen Sodalösung. Anschließend wurde unter fortgesetzter Titration 1.5 h bei pH 7.5 auf 98 - 100°C erhitzt.

Die Rohlösung wurde in der in DE-C 32 34 784 , Beispiel 2 angegebenen Weise durch Druckpermeation bis auf einen Restgehalt von 0,5% Kochsalz entsalzen und dann auf ein Gewicht von 0,8 kg aufkonzentriert. Man erhielt eine Flüssigeinstellung mit einer spezifischen Extinktion von 100 bei 350 nm, 22% Aktivsubstanz und einem Elektrolytgehalt von <0,5%.

Die Aktivsubstanz entspricht der Verbindung

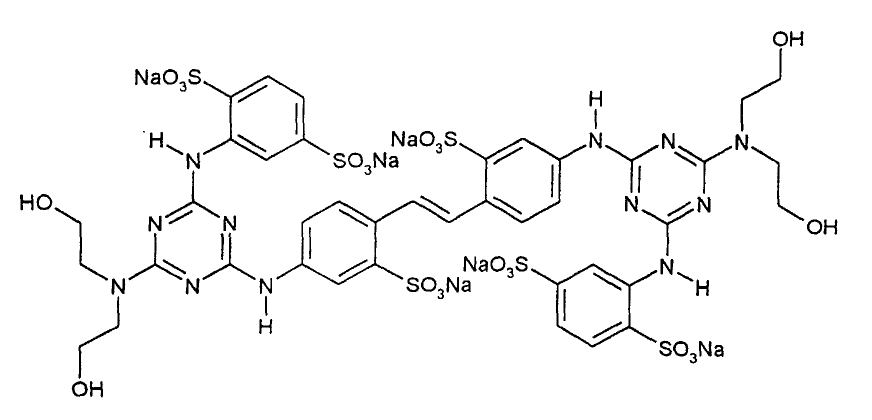

## Patentansprüche

1. Verfahren zur Herstellung von Verbindungen der Formel (I) 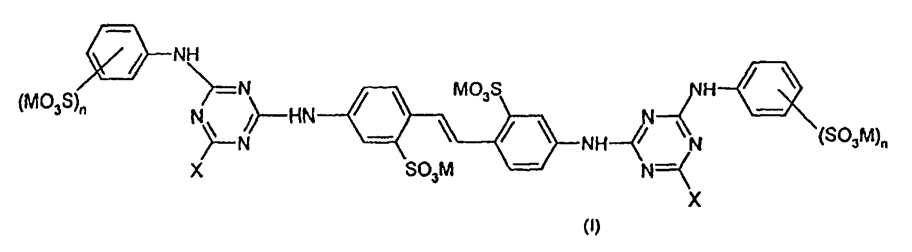 worin
n für 0, 1 oder 2 steht,
M für Wasserstoff-, ein Alkalimetallion oder ein gegebenenfalls substituiertes Ammoniumion und
X für Anilino, N-Alkylamino oder N,N-Dialkylamino steht, wobei die gegebenenfalls substituierten Alkylreste in N-Alkylamino und N,N-Dialkylamino gegebenfalls durch ein Heteroatom aus der Reihe O, N oder S unterbrochen sind und im Fall von N,N-Dialkyl die beiden Alkylreste gegebenenfalls gemeinsam mit dem N-Atom, an das sie gebunden sind, einen gesättigten 5- oder 6-gliedrigen Heterocyclus bilden,
durch Umsetzung einer Verbindung der Formel (IV) 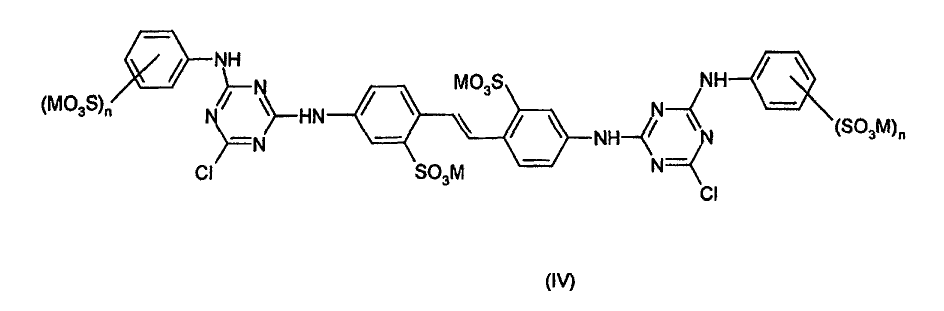 worin M und n die obengenannte Bedeutung haben,
mit einem Amin der Formel (V)
X-H (V)
worin X die obengenannte Bedeutung besitzt,
bei einem pH-Wert von 5 - 10, gegebenenfalls in Gegenwart eines von V verschiedenen Säurefängers, **dadurch gekennzeichnet, daß** man einem wäßrigen Reaktionsmedium mit einer Temperatur von wenigstens 40°C die Verbindung der Formel (IV) zugibt und daß man das Amin der Formel (V) und gegebenenfalls den Säurefanger unabhängig voneinander vor und/oder während der Zugabe von IV dem wäßrigen Reaktionsmedium zugibt.

2. Verfahren gemäß Anspruch 1, **dadurch gekennzeichnet, daß** das wäßrige Reaktionsmedium eine Temperatur von 60 bis 140°C, insbesondere 80 bis 100°C besitzt.

3. Verfahren gemäß Anspruch 1, **dadurch gekennzeichnet, daß** die Umsetzung bei einem pH-Wert von 6 bis 9, insbesondere von 7 bis 8 erfolgt.

4. Verfahren gemäß Anspruch 1, **dadurch gekennzeichnet, daß** die Verbindung der Formel IV als wäßrige Lösung oder Suspension eingesetzt wird.

5. Verfahren gemäß Anspruch 4, **dadurch gekennzeichnet, daß** die wäßrige Lösung oder Suspension von IV das Amin der Formel V bereits ganz oder teilweise enthält.

6. Verfahren gemäß Anspruch 1, **dadurch gekennzeichnet, daß** die Temperatur des Reaktionsmediums wenigstens 20°C höher ist als die der zuzugebenden Verbindung IV.

7. Verfahren gemäß Anspruch 1, **dadurch gekennzeichnet, daß** als der von der Verbindung V verschiedene Säurefänger ein Alkalihydroxid, Alkalicarbonat, Alkalihydrogencarbonat oder tert.- Amin eingesetzt wird.

8. Verfahren gemäß Anspruch 1, **dadurch gekennzeichnet, daß** der Säurefänger in Abhängigkeit vom pH-Wert automatisch zudosiert wird.

9. Verfahren gemäß Anspruch 1, **dadurch gekennzeichnet, daß** n für 0 steht.

## Claims

1. Process for the preparation of compounds of the formula (I) 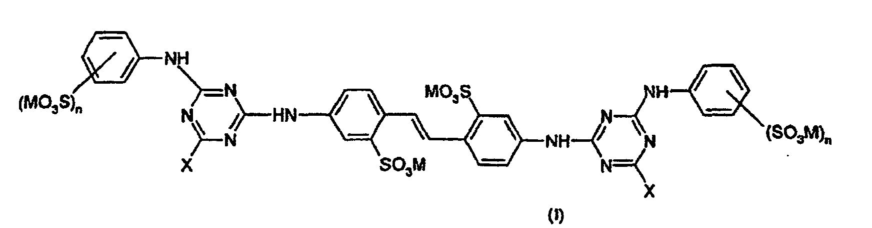 in which
n represents 0, 1 or 2,
M represents hydrogen, an alkali metal ion or an optionally substituted ammonium ion and
X represents anilino, N-alkylamino or N,N-dialkylamino, the optionally substituted alkyl radicals in the N-alkylamino and N,N-dialkylamino optionally being interrupted by a hetero atom from the series consisting of O, N and S, and, in the case of N,N-dialkyl, the two alkyl radicals optionally forming, together with the N atom to which they are bonded, a saturated 5- or 6-membered heterocyclic ring,
by reaction of a compound of the formula (IV) 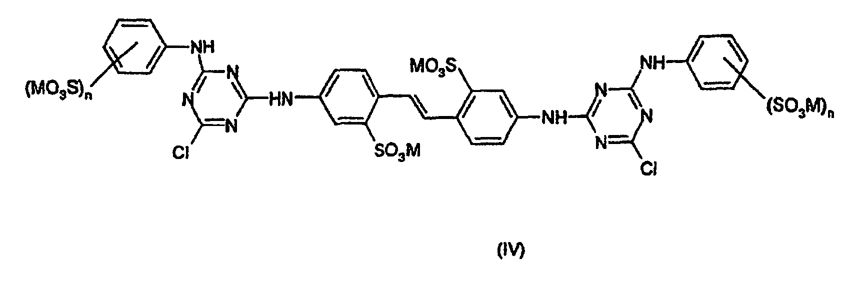 wherein M and n have the abovementioned meaning,
with an amine of the formula (V)
X-H (V)
wherein X has the abovementioned meaning,
at a pH of 5 - 10, if appropriate in the presence of an acid-trapping agent which differs from V, **characterized in that** the compound of the formula (IV) is added to an aqueous reaction medium with a temperature of at least 40°C, and **in that** the amine of the formula (V) and, if appropriate, the acid-trapping agent are added to the aqueous reaction medium independently of one another before and/or during the addition of IV.

2. Process according to Claim 1, **characterized in that** the aqueous reaction medium has a temperature of 60 to 140°C, in particular 80 to 100°C.

3. Process according to Claim 1, **characterized in that** the reaction is carried out at a pH of 6 to 9, in particular 7 to 8.

4. Process according to Claim 1, **characterized in that** the compound of the formula IV is employed as an aqueous solution or suspension.

5. Process according to Claim 4, **characterized in that** the aqueous solution or suspension of IV already comprises all or some of the amine of the formula V.

6. Process according to Claim 1, **characterized in that** the temperature of the reaction medium is at least 20°C higher than that of the compound IV to be added.

7. Process according to Claim 1, **characterized in that** an alkali metal hydroxide, alkali metal carbonate, alkali metal bicarbonate or tertiary amine is employed as the acid-trapping agent which differs from the compound V.

8. Process according to Claim 1, **characterized in that** the acid-trapping agent is metered in automatically as a function of the pH.

9. Process according to Claim 1, **characterized in that** n represents 0.

## Revendications

1. Procédé pour la préparation de composés de formule (I) 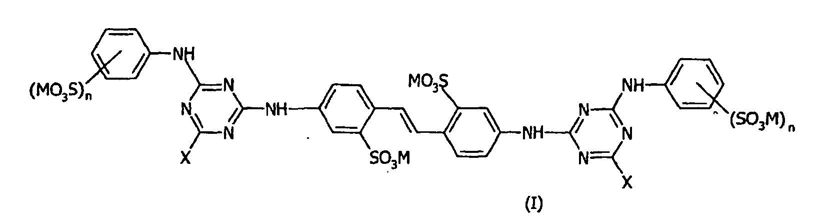 dans laquelle
n est égal à 0, 1 ou 2,
M représente l'hydrogène, un ion de métal alcalin ou un ion ammonium éventuellement substitué et
X représente un groupe anilino, N-alkylamino ou N,N-dialkylamino dans lesquels les groupes alkyle éventuellement substitués sont éventuellement interrompus par un hétéroatome du groupe formé par O, N ou S et, dans le cas de groupes N,N-dialkyle, les deux groupes alkyle peuvent le cas échéant former un hétérocycle saturé à 5 ou 6 chaînons avec l'atome d'azote auquel ils sont reliés,
par réaction d'un composé de formule (IV) 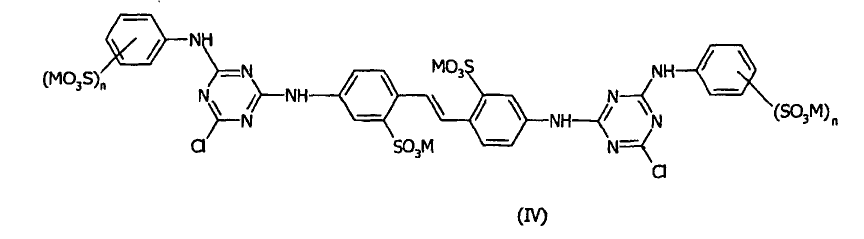 dans laquelle M et n ont les significations indiquées ci-dessus,
avec une amine de formule (V)
X-H (V)
dans laquelle X a les significations indiquées ci-dessus,
a un pH de 5 à 10, éventuellement en présence d'un capteur d'acide autre que V, ce procédé se caractérisant en ce que l'on ajoute le composé de formule (IV) à un milieu de réaction aqueux à une température d'au moins 40°C et on ajoute au milieu de réaction aqueux l'amine de formule (V) et le cas échéant le capteur d'acide, indépendamment l'un de l'autre, avant et/ou durant l'addition de IV.

2. Procédé selon la revendication 1, **caractérisé en ce que** le milieu de réaction est à une température de 60 à 140°C, plus spécialement de 80 à 100°C.

3. Procédé selon la revendication 1, **caractérisé en ce que** la réaction est réalisée à un pH de 6 à 9, plus spécialement de 7 à 8.

4. Procédé selon la revendication 1, **caractérisé en ce que** le composé de formule IV est mis en oeuvre à l'état de solution ou suspension aqueuse.

5. Procédé selon la revendication 4, **caractérisé en ce que** la solution ou suspension aqueuse de IV contient déjà l'amine de formule V en totalité ou en partie.

6. Procédé selon la revendication 1, **caractérisé en ce que** la température du milieu de réaction est supérieure d'au moins 20°C à celle du composé IV qui doit être ajouté.

7. Procédé selon la revendication 1, **caractérisé en ce que** l'on utilise en tant que capteur d'acide autre que le composé V un hydroxyde alcalin, un carbonate alcalin, un bicarbonate alcalin ou une amine tertiaire.

8. Procédé selon la revendication 1, **caractérisé en ce que** le capteur d'acide est ajouté automatiquement en fonction du pH.

9. Procédé selon la revendication 1, **caractérisé en ce que** n est égal à 0.
